**Europäisches Patentamt**

**European Patent Office**

**Office européen des brevets**

(19)

(11) Publication number : **0 316 428 B1**

## EUROPEAN PATENT SPECIFICATION

(12)

(45) Date of publication of patent specification :
07.04.93 Bulletin 93/14

(51) Int. Cl.$^5$ : **A61M 3/00**

(21) Application number : **88905325.2**

(22) Date of filing : **31.05.88**

(86) International application number :
**PCT/US88/01824**

(87) International publication number :
**WO 88/09677 15.12.88 Gazette 88/27**

(54) **HYPODERMIC FLUID DISPENSER.**

(30) Priority : **08.06.87 US 59620**

(43) Date of publication of application :
**24.05.89 Bulletin 89/21**

(45) Publication of the grant of the patent :
**07.04.93 Bulletin 93/14**

(84) Designated Contracting States :
**AT BE CH DE FR GB IT LI LU NL SE**

(56) References cited :
**DK-A- 0 087 314**
**US-A- 1 880 354**
**US-A- 2 762 369**
**US-A- 2 800 903**
**US-A- 3 138 157**
**US-A- 3 262 449**

(56) References cited :
**US-A- 3 419 007**
**US-A- 3 557 784**
**US-A- 4 396 384**
**US-A- 4 518 385**
**US-A- 4 552 277**
**US-A- 4 592 742**

(73) Proprietor : **D'ANTONIO, Nicholas F.**
**7695 Admiral Drive**
**Liverpool NY 13088 (US)**

(72) Inventor : **D'ANTONIO, Nicholas F.**
**7695 Admiral Drive**
**Liverpool NY 13088 (US)**

(74) Representative : **Cheyne, John Robert**
**Alexander Mackenzie**
**HASELTINE LAKE & CO. 28 Southampton**
**Buildings Chancery Lane**
**London WC2A 1AT (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to apparatus for the hypodermic injection of fluids.

Hypodermic fluid dispensers fall into two broad categories, namely: needle hypodermic dispensers and jet injectors. Jet injectors are particularly useful in immunization programs which involve the delivery of a non-living virus; and needle injectors are useful in the delivery of a non-living virus and of medications.

In general, jet inoculation, as compared to needle inoculation, is less traumatic, presents a lower risk of cross contamination, requires less operator training, and allows a higher number of procedures per unit of time.

Although both needle hypodermic injectors and high pressure jet injectors have been widely used, the presently known devices have serious disadvantages. The problems associated with needle injectors in the spread of AIDS (Acquired Immune Deficiency Syndrome) alone is sufficient motivation to avoid proliferation of today's needle systems. People cannot be relied upon to dispose of needle injectors in accordance with instructions and good practice; and an element of the population are tempted to reuse needles without knowledge of or regard for safe practices.

Jet injectors generally avoid the above referenced problems associated with needle injectors; however, the presently available injectors are expensive; prone to failure; too bulky to transport conveniently; and are generally inconvenient to use.

In GB-A-789029 there is described a hypodermic needle syringe, which is primed for use and which comprises a tubular body having a hypodermic needle slidably mounted in the tubular body, a central fluid containing compressible ampoule which can be perforated by the hypodermic needle and a compressed spring mounted behind the ampoule which when released causes perforation of the ampoule by the needle and injection of the fluid out through the needle. After use the needle remains extended out of the tubular body.

In US-A-3419007 there is described a collapsible ampoule for use with a needleless hypodermic injector (i.e. a jet injector), which ampoule is made of metal.

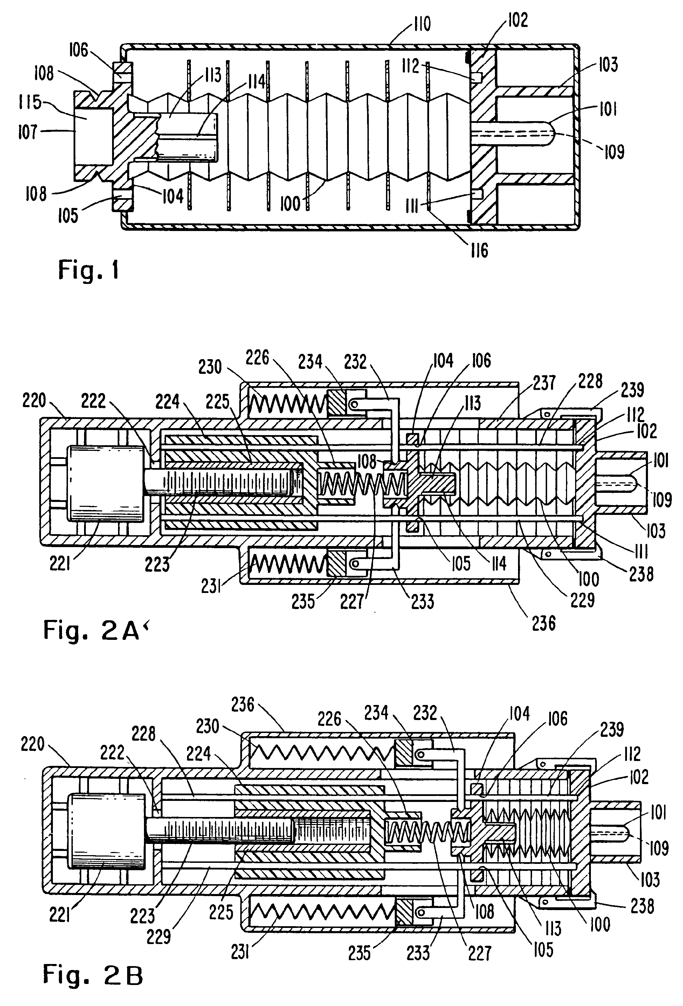

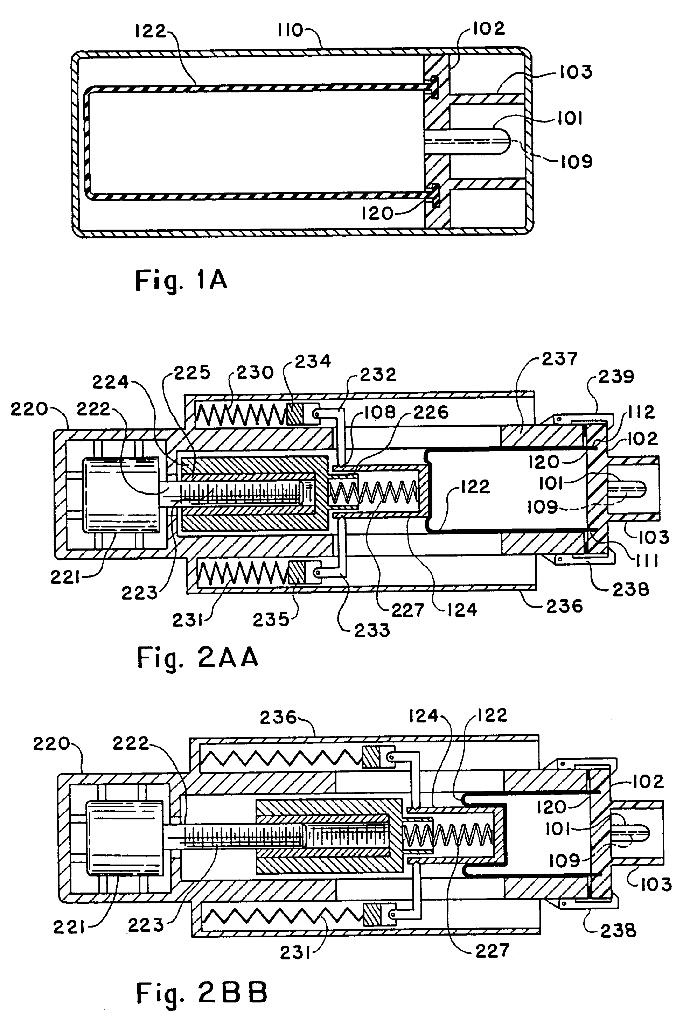

According to the present invention there is provided a hypodermic fluid dispensing system capable of receiving a fluid dispenser (Fig. 1, 1A, 5A or 8A) having a collapsible tubular body (100, 122, 500 or 804) which forms a chamber capable of containing a hypodermic fluid and a fluid output port (109, 508, or 807)

capable of communicating with the chamber of the body which system is provided with;

means for applying pressure (223, 227 + 113; 510 + 511; or 703) to one end of the body of the dispenser so as to discharge fluid contained in the chamber through the output port,

characterized in that the system is also provided with a motor (221, or 1) for driving the means for applying pressure to the dispenser.

The dispenser may be a jet injector which comprises a collapsible tubular body (100, 122 or 804) which forms a chamber and a nozzle (101 or 806) provided with a fluid output port (109, 508 or 807) capable of communicating with the chamber of the body and which jet injector is either reusable or disposable.

Alternatively the dispenser may be a jet injector which comprises a nozzle (806)provided with a fluid output port (807) and a collapsible tubular body (804) which forms a sealed chamber and which is slidably mounted with respect to the nozzle, wherein means (808) are associated with the nozzle for breaching the sealed chamber to allow fluid contained therein to pass out of the output port.

The output port may be provided in a replaceable nozzle.

The fluid output port (508) may be in the form of a hypodermic needle (506) capable of communicating with the chamber of the body; and the dispensing system may then further comprises means of covering the needle (504) before and after dispersing of the fluid.

Advantageously, the first aspect of the present invention is applicable to both needle injectors and high pressure jet hypodermic injectors.

The invention is illustrated in the accompanying drawing in which:

Figs. 1 and 1A illustrate two multiple inoculation jet injectors in accordance with the present invention;

Figs. 2a and 2aa illustrate jet injector systems with fresh injectors of Figs. 1 and 1A installed therein;

Figs. 2b and 2bb illustrate the jet injector systems of Figs. 2a and 2aa with the injectors partially expended;

Fig. 3 and Fig. 4 are a schematic representation of the control and monitoring circuitry of the jet injector of Fig. 2;

Figs. 5a through 5d illustrate a needle hypodermic injector in accordance with the present invention in a sequence of stages which occur in use of the injector;

Fig. 6 is a block diagram overview of a multiple inoculation jet injector system in accordance with the present invention;

Fig. 7 is an alternative embodiment of a jet injector in accordance with the present invention; and

Fig. 8A and 8B illustrate the details for the serum cartridge of Fig. 7 jet injector.

Fig. 1 illustrates a liquid filled jet injector stored in a protective cover 110. The jet injector of Fig. 1 comprises:

a liquid filled bellows 100; a front plate 102 which comprises: a pair of guide rod recesses 111 and 112, a set of thin washers 116 which have an inner diameter slightly larger than the minor diameter of bellows 100, a pair of holes in each of the washers 116 that are aligned with the guide rod recesses 111 and 112 and with the openings 105 and 106; a spacing guard ring 103, a nozzle 101 with an output port 109; a ram 113 with a groove 114; and a back plate 104. The back plate comprises openings 105 and 106, aligned with holes 117 in washers 116, which accept a pair of guide rods when the injector is installed in the system of Figs. 2a and 2b; and a ring 115 which forms the recess 107 for receiving a drive spring 227 of the system of Fig. 2a. The injector of Fig. 1 may be a disposable injector or a permanent reusable injector and the output port may be removable and replaceable or a permanent part of the bellows. In either case, device economy is improved by fabricating a moulded output port having the flow orifice formed by first inserting a performed metal, or other sufficiently sturdy member, whose size and shape i.e., length, diameter and flow path angulation are chosen to provide laminar flow. Some of the other possible methods for forming the orifice include laser boring, water jet cutting and electron beam cutting.

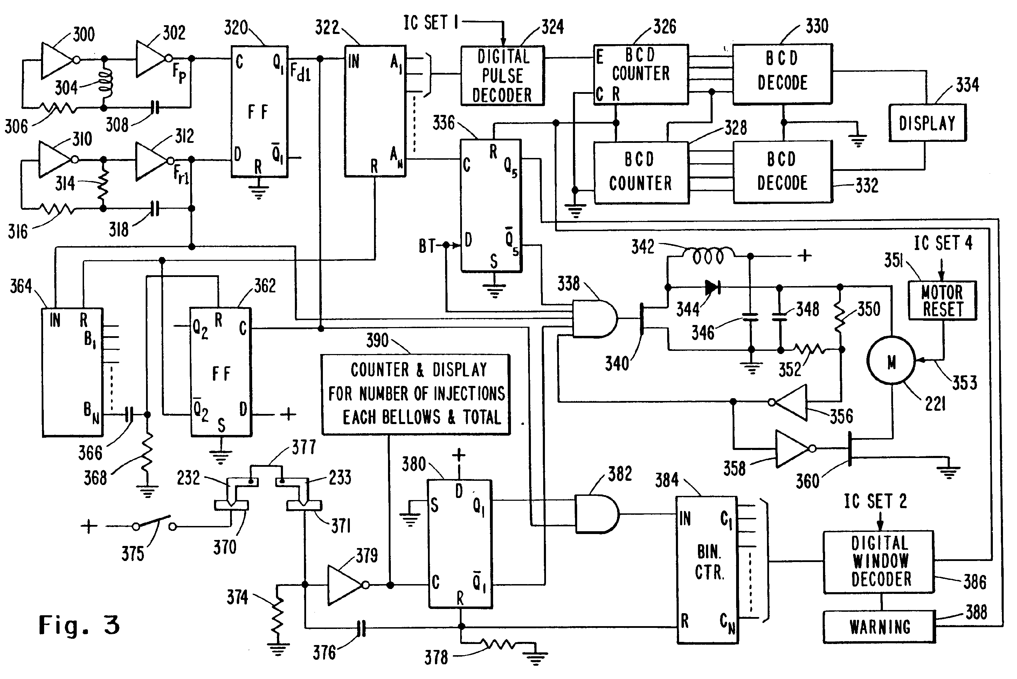

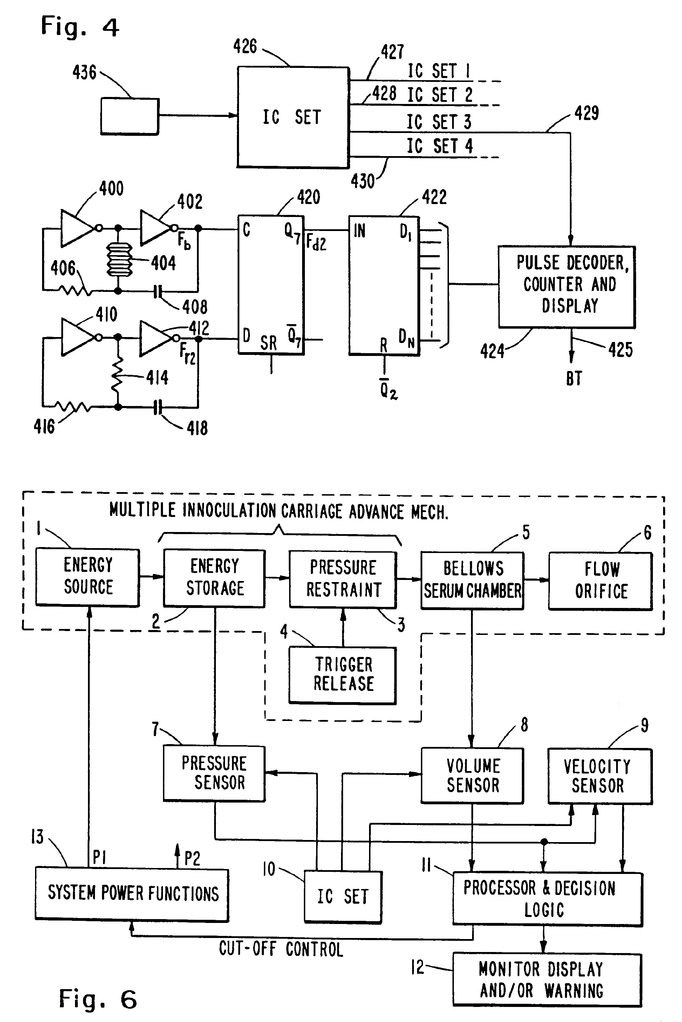

Figure 2a is a cross section representation of an illustrative embodiment of a hypodermic jet injector system with a fresh jet injector of Fig. 1 installed therein. The system of Fig. 2 in general comprises: a housing 220; the control, monitoring and display arrangements of Figs 3 and 4 (not shown in Fig. 2a) ; a battery operated drive motor 221; a motor output shaft 222 with a threaded portion 223; a loading ram 224 with internal threads 225 which mate with the threads 223; an energy storage spring 227; a reluctance transducer shield 226 to be described with respect to Fig. 3 later herein; a pair of guide rods 228 and 229 that serve to align and support the bellows 100; retaining latches 238 and 239; and a trigger mechanism which comprises the detents 232 and 233, the follower springs 230 and 231 and the follower blocks 234 and 235. The mechanism for releasing the detents 232 and 233 is not shown in Fig. 2 and any suitable mechanical linkage which effects the simultaneous lifting of the detents 232 and 233 is satisfactory.

An alternate embodiment of the injector of Figs. 1, 2a and 2b is illustrated in Figs. 1a, 2aa, and 2bb. In the alternate embodiment, the liquid filled bellows 100 is replaced by a collapsible liquid filled " hat " diaphragm type structure. Detents 111 and 112 of Fig. 1 are replaced in Fig. 1a by slot 120 for mounting and firmly securing hat structure 122 to the front plate 102.

Fig. 2aa is a cross section representation of the injector system with a fresh injector installed therein. When the diaphragm 122 is inserted in the now conformal housing 220, a slight initial collapse of 122 occurs to facilitate chamber venting and to assure an effective inner folding action as the multiple injections follow. These conformal shape of housing 220 constrains fluid chamber 122 and thus prevents undesired outward expansion under the influence of the injection pressure when the spring 227 is released. Back plate 124 is configured to assure that the detents 232 and 233 do not interfere with fluid chamber 122 as it progressively folds inside its outside diameter (Fig. 2bb ) with each additional injection.

While the following discussion is specific to the embodiment of Figs. 1,2a and 2b, the discussion applies equally to the alternate embodiment of Figs. 1a, 2aa and 2bb.

Prior to the time that a fresh jet injector of Fig. 1 is installed in the system of Fig. 2, a manually operated reset switch 436 of Fig. 4 is operated to enable the Integrated circuit (IC) Set circuit 426 of Fig. 4 to establish initial system conditions. Output signals of the circuit 426 serve to: enable motor reset 351 of Fig. 3 to draw the loading ram 224 back into the initial position illustrated in Fig. 2a; and to initialize the digital pulse decoder 324, the digital window decoder 386 and the pulse decoder, counter, and display 424. Alternatively, the ram 224 can be manually returned to the initial position at the time that a fresh bellows is installed.

The functions of the pulse decoder 324, the window decoder 386 and the pulse decoder counter and display 424 will be apparent from the description of Figs. 3 and 4 which appears later herein. After the system is thus conditioned, a fresh jet injector is removed from the protective cover 110 and inserted into the housing 220 as illustrated in Fig. 2a. To install the jet injector, the guide rods 228 and 229 are inserted into the openings 105 and 106 in the back plate 104, through the openings 117 in the washers 116, and the injector is moved into the housing 220 until the front face of the front plate 102 is clamped by the latches 238 and 239. As the jet injector is moved into the housing 220, the detents 232 and 233 engage the notches 108 in the back plate 104 and follow the motion of the back plate as it is moved to the left in Fig. 2a. The follower blocks 234 and 235 follow the motion of the detents 232 and 233 to the left and thus compress the follower coil springs 230 and 231. Further, when a fresh injector is in position as illustrated in Fig. 2a, the energy storage spring 227 enters the recess 107 in the jack side of the back plate 104. The system of Fig. 2a is in condition for an operator to perform a series of inoculations.

Under operator control, the system of Figs. 2a, 3 and 4 selectively provides power to the motor 221 to advance the ram 224 to the right in Fig. 2a and thus compress the coil spring 227. As described later herein, the circuitry of Fig. 3 monitors the degree of compression of the spring 227 and removes power from the motor when the compression reaches a target value. The target value is either a default value established by the manufacturer or a value established by an operator on the basis of experience. After the spring has been compressed to the target value, the guard 103 is held perpendicular to and against the skin at a suitable site of a subject to be injected. The outer ring surface of guard 103 may have a tooth like pattern to reduce the possibility of the ring sliding along the skin during the course of an injection. If the ring does slide during the injection the subject may receive a "jet cut" rather than a jet inoculation. The operator initiates injection by depressing a trigger, which as indicated earlier herein is not shown in the drawing. Depression of the trigger simultaneously releases the detents 232 and 233 from the recesses 108 in the back plate 104. The energy stored in the spring 227 is released and the back plate 104 is rapidly driven to partially collapse the bellows 100. As the bellows is collapsed, a desired amount of fluid is driven through the output port 109 in the projection 101. Advantageously,the use of a compressed spring as a source of energy provides a high initial pressure which reduces as the bellows 100 collapses. The size and the length of the port 109 and the pressure profile supplied by the compressed spring projects the fluid with a desired pressure profile which assures hypodermic injection of the fluid to the desired depth. The diameter and the length of the port 109 are chosen to assure laminar flow of the liquid from the chamber to the output tip. As the bellows collapse,guide washers 116 move together and prevent the bellows from bending under the influence of the high initial force of the injection.

As explained earlier herein, when a fresh injector is inserted into the housing 220, the follower springs 230 and 231 are compressed. Therefore, after the trigger is released, the follower blocks 234 and 235 and the detents 232 and 233 are driven to the right in Fig. 2a until the detents again engage the corresponding recesses 108 in the back plate 104. The follower blocks 234 and 235 may be connected to a small dashpot if a delay in this action is desired. The system of Fig. 2a is then again ready for the operator to initiate another injection.

Because the wall of the bellows 100 has a finite thickness, a fully collapsed bellows has a substantial length. The length of the ram 113 approximates the length of the collapsed bellows. Without the ram 113, valuable fluid is left in a fully collapsed bellows. The groove 114 in the ram 113 prevents the trapping of fluid in the portions of the bellows which surround the ram 113.

The above description is a general outline of the inoculation process without detailed reference to the control, monitoring and display apparatus of Figs. 3 and 4. While it is the intention to provide safe, low cost and convenient to use hypodermic injectors, also provided are measures of electronic monitoring and control not found in known jet injectors.

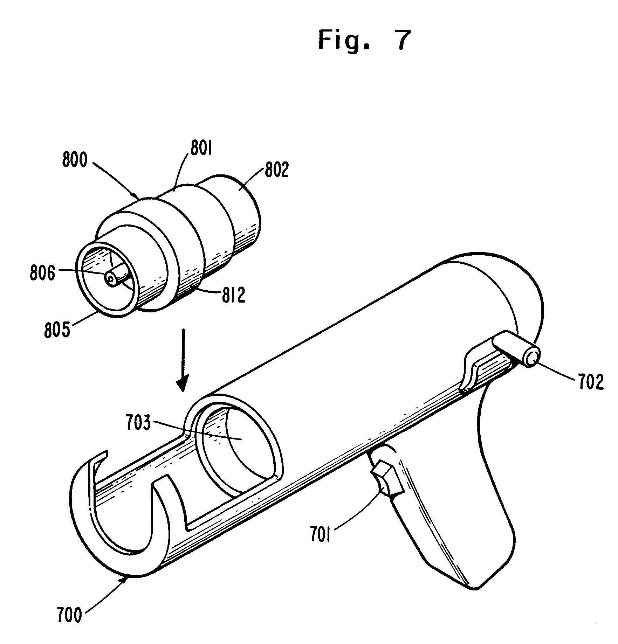

Fig. 6 provides a block diagram functional overview of a hypodermic jet injector system in accordance with the present invention. The dotted line of Fig. 6 provides a logical division between the mechanical portions of the injector system and the electronic monitoring and control portions of the system.

The functional boxes 1 through 6 of Fig. 6 correspond to elements of the illustrative embodiment of Figs. 2a and 2b as follows:

| Fig. 6 | Fig. 2a |
|---|---|
| Energy Source 1 | Motor 221 |
| Energy Storage 2 | Spring 227 |
| Pressure restraint 3 | Detents 232 & 233 |
| Trigger release 4 | Not shown in drawing |
| Bellows serum chamber 5 | Jet injector of Fig. 1 including Bellows 100 |
| Flow orifice 6 | Output port 109 |

Although the illustrative embodiment of Fig. 2a employs a battery operated geared down motor 221 to compress the coil spring 227, this requirement can be fulfilled by a variety of manual arrangements utilizing gears or other means of mechanical advantage. While the spring is preferred for storing the energy, the blocks 1 and 2 of Fig. 6 could be replaced by other arrangements e.g., a powerful solenoid. The critical requirement of the blocks 1 and 2 is that the bellows 100 receives enough force for a sufficient period of time to assure an effective inoculation.

The monitoring functions of Fig. 6 inform an operator when the device is ready to perform an injection i.e., all system parameters are within acceptable limits of performance. A warning is issued when performance is not within limits and the system is disabled in the event of a malfunction.

The pressure sensor 7 of Fig. 6 monitors the status of the energy storage device 2 and compares the magnitude of the stored energy to a target magnitude. When the magnitude of the energy stored reaches the target value, the storage of energy is terminated. The target value may be a default value established by the manufacturer or a value established by the operator on the basis of experience with different subjects e.g., adults, children, animals which may be better served with different pressures. The target pressure value is one of the " initial conditions " which an operator may set by controls in the IC Set function 10 of Fig. 6.

The volume sensor 8 provides assurances that a correct amount of liquid is used in each injection.

The velocity sensor 9 of Fig. 6 determines the time required for the stored energy to decay to equilibrium after an injection. The decay time is a measure of output port performance. If the output port is partially clogged, the pressure decays too slowly; and if the output port is worn or too large the pressure will decay too rapidly. If a failure is detected, a warning will be issued to the operator and the system is disabled until corrective action is taken.

The IC Set 10 of Fig. 6 permits an operator to select initial condition values for the pressure sensor 7, the volume sensor 8 and the velocity sensor 9.

The processor and decision logic 11 issues control signals to the system power control 13 and status signals to the monitor display and warning unit 12.

In addition to the control and monitoring function described above herein, the circuit arrangements of Fig. 4 maintain a record of the number of injections completed.

The implementation of the system functions by the arrangements of Figs. 3 and 4 will be understood from the following description.

Digital inverters 310 and 312, resistors 314 and 316, and capacitor 318 are configured to form a reference frequency oscillator. The operating frequency $F_{r1}$ is determined by the time constant of the resistor 314 and the capacitor 318.

Digital inverters 300 and 302, capacitor 308, and variable sensing inductance 304 in Fig. 3 form a variable frequency reluctance transducer oscillator which has an operating frequency $F_p$. The operating frequency of the oscillator varies as a function of the value of the inductance 304. The coil 304, which is not shown in Fig. 2a is mounted at the centre of recess 107 and inside the energy storage spring 227 and is partially covered by the reluctance shield 226 of Fig. 2a. A change in the relative position of the coil 304 and the shield 226 as the spring 227 is compressed changes the inductance of the coil 304. Accordingly, the frequency of the oscillator, which is determined by the time constant of the inductance 304 and the capacitor 308, is determined by the degree of compression of the spring 227. A reluctance transducer oscillator with a sensing inductance as described above is known from my U.S. patent application S.N. 607,654 filed May 7, 1984.

Flip flop 320 is configured as a frequency mixer which provides a digital output signal which has a pulse rate $F_{d1}$ which is the difference between the reference pulse rate $F_{r1}$ and the oscillator frequency $F_p$. In the absence of pressure on the spring 227, the frequencies $F_{r1}$ and $F_p$ are equal and the pulse rate $F_{d1}$ at the "1" output of flip flop 320 is zero.

In the illustrative embodiment of Fig. 3, the motor 221 is driven by a series of high energy, relatively high voltage pulses. The output of AND gate 338 controls the generation of the motor drive pulses. The inputs to the AND gate 338 comprise: the "0" output of the flip flop 336 which remains high until the target value of spring compression is reached; the BT conductor from pulse decoder 424; the "0" output of the flip flop 380 which is high except when the trigger is activated to initiate an injection; the output of the inverter 356 which is high until the charge on capacitor 348 reaches a critical value; and the output conductor of the reference oscillator. When enabled, the output signal of AND gate 338 turns the FET 340 on and off at the rate $F_{R1}$ of the reference oscillator. When the transistor 340 is on, current will flow from positive potential through inductance 342 and the transistor 340, to ground. When the transistor 340 is subsequently turned off, the energy stored in the magnetic field of coil 342 will discharge through the path which is comprised of diode 344 and capacitor 348. The resistors 350 and 352 are of relatively high value; therefore, very little energy is lost in the path to ground through those two resistors. The magnitude of the voltage generated by the collapse of the magnetic field of coil 342 is very high and is dictated by the rate of collapse of the field. The rate of collapse is determined by the impedance of the discharge path. The diode 344 prevents reverse flow of current due to the build up of voltage on the capacitor 348. Capacitor 346 is a stabilizing capacitor which provides an extra measure of current for the coil 342 during the ON state of transistor 340. When the charge on capacitor 348 reaches a predetermined value the output of the threshold detector 356 will go low and gate 338 is disabled. The predetermined value represents a charge large enough to advance the motor 221. When the output of detector 356 goes low, the output of inverter 358 goes high to enable transistor 360 to provide a path for discharging the capacitor 348 through the winding of motor 221. When the charge on the capacitor falls below the threshold value of detector 356, the output of detector 356 goes high to enable gate 340 to initiate another cycle of charging capacitor 348; and the output of inverter 358 goes low to disable transistor 360. Charging cycles will continue until flip flop 336 is set to the "1" state which indicates that the energy stored in spring 227 has reached the target value. In the drawing, the output labeled $Q_5$ is the "1" output of the flip flop 336 and the complement output is termed the "0" output herein.

Flip flop 336 is controlled by the $A_n$ output conductor of counter 322, by an output signal of the digital window decoder 386, and by the BT conductor. The flip flop 336 is set to the "1" state when the $A_n$ output of the counter 322 goes high if the BT conductor is high; and is reset by the output conductor of the decoder 386. Counter 322, in turn, is controlled by the $F_{d1}$ signal at the output of flip flop 320 and by the output of flip flop 362. Flip flop 362 is set by a $F_{d1}$ signal at the output of flip flop 320 and reset by a $B_n$ output signal of counter 364. Counter 364 defines a period of time in terms of pulses of the reference frequency $F_{r1}$ and counter 322 counts the difference frequency pulses $F_{d1}$. Since counter 364 and counter 322 are reset at the same time by an output signal of flip flop 362, counter 364 provides a measurement window of time which runs from reset time to the next reset time. The $A_n$ output conductor will remain low until the deformation of the spring 227 reaches the target value. When the counter 322 reaches the $A_n$ count within the measurement time window, flip flop 336 is set and gate 338 is disabled. At the same time, the "1" output of flip flop 336 is transmitted to the warning function 388 to indicate that the device is ready for an injection procedure. Flip flop 336 can be set only if the "BT" input to the D terminal of that flip flop is high. As will be explained with respect to Fig. 4, the BT conductor will be high if the bellows test is satisfactory. The digital code which is stored in counter 322 during a measurement time interval corresponds to the instant deformation of the energy storage spring 227. The digital pulse decoder 324, in response to the digital code in counter 322, generates input signals for the BCD counters 326,328. For example, if the deformation of the spring which is equivalent to one pound of force on the spring provides ten cycles of differential frequency $F_{d1}$, decoder 324 will convert the code in counter 322 to a single pulse for BCD counters 326,328. With a count of one in the counters 326,328, the BCD decoders 330,332 provide signals to the display 334 to display the value, one pound. Any number can be displayed with appropriate decoding by pulse decoder 324. By virtue of the display 334, the operator knows that the appropriate level of energy is stored in the spring 227 and that an injection may be initiated. The flip flop 336 remains set until an injection has been successfully completed. If the velocity test fails, a warning in 388 will issue and flip flop 336 will not be reset. Accordingly, remedial action must be taken before preparation for another injection can be started.

The power on switch 375 in the lower left portion of Fig. 3, connects positive battery potential to the input of inverter 379 through the contact segments 370 and 371, detents 232 and 233, and link 377. The contact segments 370 and 371 lie in the recesses 108 on the back plate 104 shown in Fig. 1 and in Fig. 2. When the trigger is operated, the detents 232 and 233 are disconnected from the contact segments 370 and 371; and because the input is referenced to ground through resistor 374, the output of inverter 379 goes high. A high signal from the output of inverter 379 increments a counter in 390 to display the number of injections completed from the current bellows; and causes the "D" type flip flop 380 to be set to the "1" state. Consequently the "0" output of flip flop 380 goes low which disables AND gate 338. The high signal on the "1" output of flip flop 380 enables AND gate 382 to pass $F_{d1}$ difference frequency signals to the input of counter 384. As explained earlier herein the difference frequency will reduce to zero when the spring 227 comes to equilibrium after an injection. The count which is accumulated in the counter 384 is thus representative of the time required for the bellows to be partially collapsed. The window decoder 386 evaluates the count in the counter 384 on the basis of the expected values established by IC 2. If the count is larger than the expected limits, it is probable that the output port is plugged and if the count is smaller than the expected limits it is probable that the output port is enlarged beyond acceptable limits. In either event, a warning signal is displayed by the warning indicator 388 and the flip flop 336 will not be reset until remedial action is taken. If the count in counter 384 is within limits, an output signal of digital decoder 386 will reset flip flop 336 and the BCD counters 326 and 328. When that occurs, the cycle to drive the motor to load energy into the spring 227 will again start. The time required for the bellows to partially collapse is short compared to the time required for the detents 232 and 233 to again settle in the recesses 108 and reconnect positive potential to the input of inverter 379. This time relationship is positively assured if a dashpot is employed to slow the return as suggested earlier herein. When the positive potential reappears at the input of inverter 379, capacitor 376 and resistor 378 which are configured as a high pass filter, produce a reset pulse to flip flop 380 and counter 384 in preparation for the next injection. In the event that a very large volume injection is to be performed, the time required to inject the fluid may exceed the time for the detents 232 and 233 to settle in recess 108. In that case the illustrative high pass reset circuitry can be replaced with circuitry with appropriate delay.

Fig. 4 provides an arrangement for testing the integrity of the liquid filled bellows 404. Inverters 400 and 402 are configured as an oscillator in which the output frequency $F_b$ is determined by the impedance across the entire bellows 404. Inverters 410 and 412 are configured as a fixed frequency oscillator having a frequency $F_{r2}$; and flip flop 420 is connected as a frequency mixer for the signals $F_{r2}$ and $F_b$. In the configuration of Fig. 4, the collapsing bellows behaves as a variable resistance; therefore, the frequency of the mixer output signal $F_{d2}$ is minimum when the bellows is full. As the bellows collapses, the impedance decreases and the differential signal $F_{d2}$ increases. The counter 422 accumulates the $F_{d2}$ signals during a measurement time interval defined by the "0" output conductor of flip flop 362 of Fig. 3; and pulse decoder and display 424 displays bellows status information. The use of the time period provided by the Dn count is for purposes of illustration. In the event that a different time period is desired, additional counter outputs and flip flops are provided. The arrangements of 424 evaluate the interval count in counter 422 on the basis of the IC set 3 information which defines a range or window of acceptable values. If the count falls within the range of acceptable values, a high BT signal will be generated and flip flop is set on occurrence of the next succeeding $A_n$ signal from counter 322. However, if the serum within the bellows has excessive voids, clots or an incorrect consistency for some reason or another, the $F_b$ frequency will fall outside the acceptable range and the count in 422 will fall outside the preselected window of performance.

It should be noted that fluid may be used as a dielectric material in an alternative embodiment in which a variable capacitance determines the frequency $F_b$. In that embodiment the variable bellows is located at the position of the capacitor 408 and a fixed resistor placed at the position 404 in Fig. 4. In this case, the two ends of the bellows form the capacitor plates and the serum fluid is the dielectric material. As the length of the bellows decreases, the capacitance increases and the frequency $F_b$ decreases.

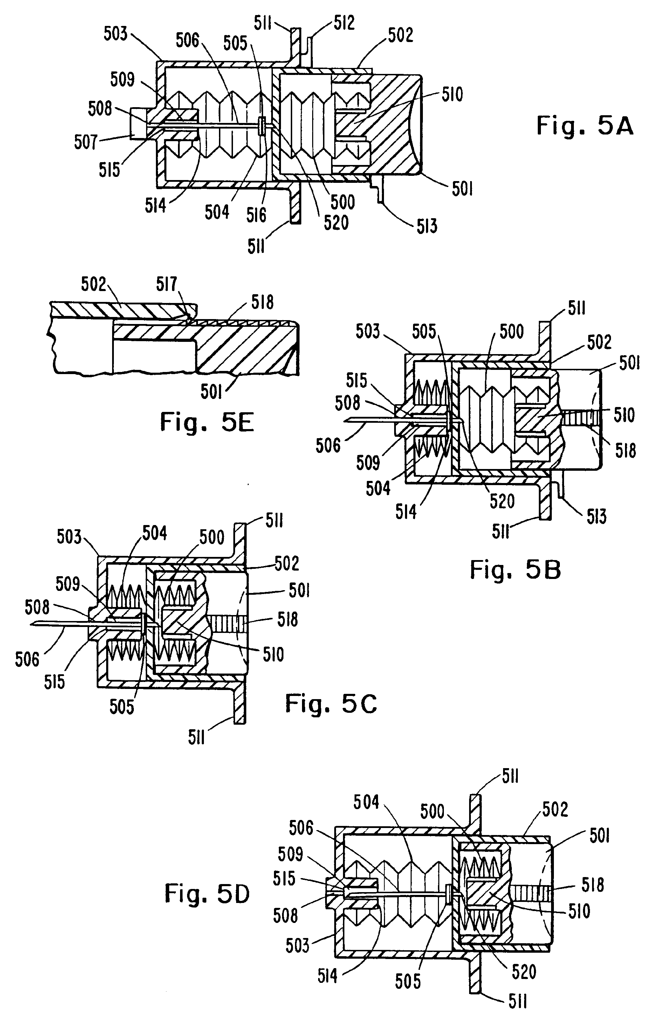

Figs. 5a to 5d show a needle type hypodermic injector in accordance with the present invention in various stages in the use of the injector. The injector of Fig. 5a comprises a bellows 500 sealed with end cap and ram 510; a front housing 503, a rear housing 502; a pressure piston 501; a needle output port 508 with a flange 511; a bellows shaped needle sheath 504; and removable cap 507. Fig. 5a illustrates a fresh injector prior to use. As in the injector embodiment of Figs. 1 and 2, the support guide rings 116 of those figures may be employed in the embodiment of Figs. 5a to 5d. The bellows 500 may contain a liquid serum or a lyophilized (freeze dried) vaccine. In the latter case a liquid which is stored in the sheath bellows 504 is driven into the bellows 500 as the needle is exposed as described below herein.

The bellows 500, the rear housing 502 and the front housing 503 all may be fabricated of clear plastic material so that the operator can observe whether or not blood is drawn into the bellows 500 when the pressure piston is slightly withdrawn.

The break away seal 512 and the cap 507 are removed to permit the operator to expose the needle 506. The need to remove the cap 507 may be eliminated if the cap 507 is made of a self sealing material e.g., pure latex rubber. The needle is exposed by applying pressure on the flange 511 and the pressure piston 501 to draw them together. Typically, the thumb is placed on the pressure piston 501 and index finger and the adjacent finger are placed on the flange 511. The resistance of the sheath bellows 504 is sufficient to cause the bellows to expand after use; however, the resistance of the bellows 504 is small compared to the force required to compress the liquid bellows 500 to eject the liquid through the needle output port. Therefore as pressure is applied between the flange 511 and the pressure piston 501 the sheath bellows will collapse to expose the needle. As the sheath bellows is collapsed the surface 514 engages the needle flange 505 and the needle moves to the right to breach the wall of the liquid bellows 500. As an option, a foam ring 516 positioned behind the flange 505 provides an additional amount of resistance to assure that the needle is fully exposed before the membrane in the serum chamber is breached by the needle. The membrane in 500 also can be made of self-sealing latex diaphragm material which will tend to hold the needle in place after the injection is completed and the sheath is extended to cover the needle. The first intermediate state of the injector is illustrated in Fig. 5b.

In the cases where the serum is stored in the bellows in a liquid state, the bellows 504 can be replaced with a simple coil spring. However, if a suggested earlier herein, the vaccine is stored in the lyophilized state, the fluid required to turn the vaccine to the liquid state is stored in the bellows 504. In this latter case, the liquid in bellows 504 is forced into bellows 500 through a hole in the membrane of the bellows 500 which is breached when the bellows 504 is compressed to expose the needle 506.

After the needle is inserted into the injection site, pressure is applied between the pressure piston 501 and the flange 511 to collapse the liquid bellows 500 and eject fluid through the output into the injection site. The state of the injector after depletion of the injection fluid is illustrated in Fig. 5c. As shown in Fig. 5e, a sawtooth pattern 518 on the outer surface of the member 501 and a single sawtooth 517 on the inner surface of the pressure piston 502 permit the pressure piston 501 to be advanced into the member 502 and thus compress the bellows 500.

However, the cooperation of 517 and 518 prohibits withdrawal of the piston 501 after engagement of 517 and 518. As an option, the end of the ram 510 is shaped to strike and crush the end 520 of the needle 506 when the bellows 500 is fully collapsed. This will further assure that the needle injector cannot be reused and will tend to retain the needle in engagement with the bellows 500 when the sheath bellows 504 and the sheath 503 are extended to cover the needle.

After the needle is removed from the subject and pressure between 501 and 511 is removed, the bellows 504 expands as shown in Fig. 5d. As seen in Fig.5d, when the needle sheath bellows 504 extends to its full length, the needle 506 is withdrawn from the needle guide 508. This occurs because in preparation for the injection the needle was moved to the right to breach the liquid chamber. Because the needle guide opening 508 is small compared to the trap opening 509, it is difficult if not impossible to again collapse the bellows 504 without the end of the needle 506 hitting the end wall 515 of the trap section 509. The tendency of the needle to hit the wall 515 can be enhanced by imparting a small bend in the needle 506 prior to initial installation into the guide opening 508.

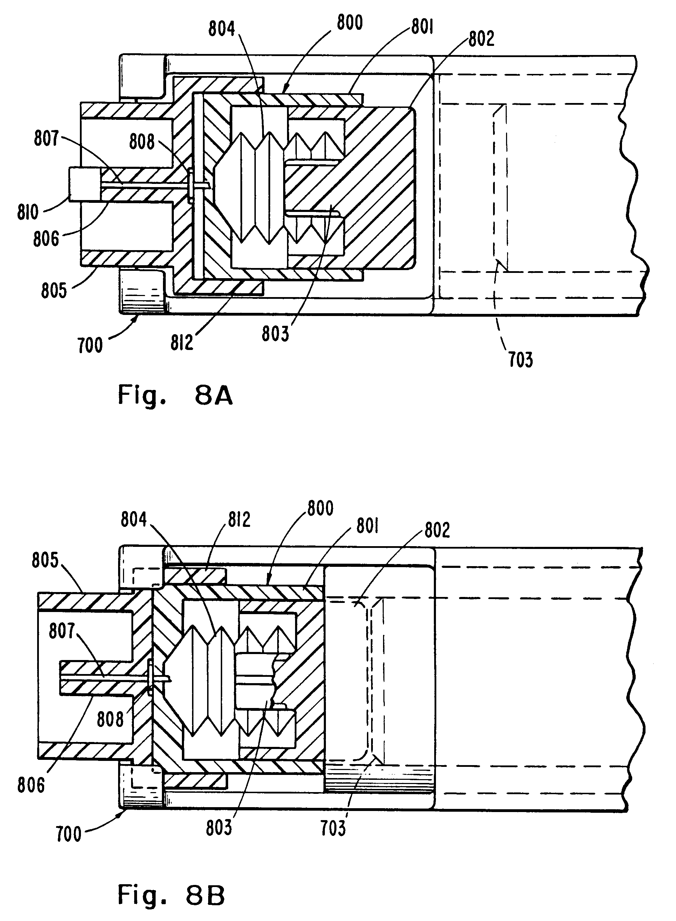

Fig. 7 shows an alternative embodiment of a jet injector system and a cartridge for that system. Details of the cartridge are shown in Figs. 8a and 8b. The system of Fig. 7 can be proportioned to handle single shot or multiple shot cartridges. The lever 702 is drawn to the rear to permit a used cartridge to be removed and a fresh cartridge installed. After a fresh cartridge is installed, the lever 702 is moved forward, to the left in Fig. 7. Any suitable driving force can be utilized to drive the pressure piston forward to collapse the bellows 804 in the cartridge 800.

The cartridge of Fig. 8a comprises a sealed liquid bellows 804 with a ram 803; a rear housing 801; a pressure piston 802; a front housing 812 which comprises a guard ring 805 and a jet output port 807 with a flange 808; and removable cap 810. Fig. 8a illustrates a fresh injector installed in the system of Fig. 7 prior to breaching the seal of the liquid bellows 804.

Fig. 8b illustrates the arrangements of Fig. 8a after the lever 702 of Fig. 7 is moved forward to breach the seal of the bellows 804 and to bring the injector piston 703 in position to drive the pressure piston 802 forward to collapse the bellows 804.

The illustrative embodiments of the invention which are disclosed herein are but representative of the invention and many changes in form and function can be made without departing from the scope of the invention, as set out in the accompanying claims.

## Claims

1. A hypodermic fluid dispensing system capable of receiving a fluid dispenser (Fig. 1,1A,5A or 8A) having a collapsible tubular body (100,122,500 or 804) which forms a chamber capable of containing a hypodermic fluid and a fluid output port (109,508 or 807) capable of communicating with the chamber of the body which system is provided with;

   means for applying pressure (223,227+113;510+511; or 703) to one end of the body of the dispenser so as to discharge fluid contained in the chamber through the output port,

   characterized in that the system is also provided with a motor (221 or 1) for driving the means or applying pressure to the dispenser.

2. A hypodermic fluid dispensing system according to claim 1, wherein the chamber is initially sealed, the output port is isolated from the chamber, and the system further comprises means for selectively bringing the output port into communication with the chamber.

3. A hypodermic fluid dispensing system according to claim 1 or 2, wherein the body is a bellows shaped collapsible tubular body.

4. A hypodermic fluid dispensing system according to any preceding claim, wherein the means for applying pressure comprises means for repeatably storing energy and means for selectively releasing that energy.

5. A hypodermic fluid dispensing system according to claim 4, wherein the means for repeatably storing energy comprises a spring and means for deforming the spring to store energy therein.

6. A hypodermic fluid dispensing system according to claim 5, wherein the spring is a coil spring and the means for deforming the spring compresses the spring.

7. A hypodermic fluid dispensing system according to any one of claims 4, 5 or 6, wherein the system comprises means for monitoring the amount of energy stored and means for terminating further storage when it reaches a predetermined value.

8. A hypodermic fluid dispensing system according to claim 7, which further comprises manually adjustable means for establishing the predetermined value.

9. A hypodermic fluid dispensing system according to claim 7 or 8, wherein the means for monitoring the energy stored comprises a source of reference pulse signals having a first pulse rate and a source of monitor pulse signals having a monitor pulse rate directly related to the magnitude of the amount of energy stored.

10. A hypodermic fluid dispensing system according to claim 9, wherein the means for repeatably storing energy is a spring and the means for monitoring the energy stored monitors the deformation of the spring and wherein the pulse rate of the monitor pulse signals is zero when the spring is at rest and is essentially not deformed.

11. A hypodermic fluid dispensing system according to claim 9 or 10, wherein the source of monitor pulse signals is provided with a variable inductance for setting the pulse rate of the monitoring pulse signals and the inductance can be adjusted as a function of the amount of energy stored.

12. A hypodermic fluid dispensing system according to any one of claims 8 to 11, which further comprises means for displaying values which define the magnitude of the stored energy.

13. A hypodermic fluid dispensing system according to any one of claims 4 to 12, which further comprises means for generating an injection pulse each time the stored energy has been released, means for counting the injection pulse, and means responsive to output signals of the counting means for displaying the number of times the energy has been released.

14. A hypodermic fluid dispensing system according to any preceding claim, which further comprises means for monitoring the performance of the system.

15. A hypodermic fluid dispensing system according to claim 14, wherein the means for monitoring the system comprises timing means, means for defining and storing parameters of acceptable system performance, means for evaluating system performance in terms of the stored parameters, and means for displaying indications of system performance.

16. A hypodermic fluid dispensing system according to claim 15, wherein the means for monitoring the system further comprises means for inhibiting system operation when the system performance falls outside the acceptable range of the stored parameters.

17. A hypodermic fluid dispensing system according to claim 15 or 16, wherein the stored parameters comprise a range of acceptable times for completion of an injection and the monitoring means comprises means for measuring the time required to complete each injection and means for evaluating the measured time in terms of the range of acceptable times.

18. A hypodermic fluid dispensing system according to claim 15 or 16, wherein the stored parameters comprise a range of values which define an expected change in length of the tubular body during each release of fluid, and the monitoring means comprises means for measuring the changes in length of the tubular body during each release of fluid, and means for evaluating the measured changes in terms of the stored parameters.

19. A hypodermic fluid dispensing system according to any preceding claim, wherein the system is formed of such a material that any contents of the chamber of the body are visible.

20. A hypodermic fluid dispensing system according to any preceding claim, which further includes means for inserting the collapsible body into the system.

21. A hypodermic fluid dispensing system according to any preceding claim, which further includes electrical storage means for activating the motor.

22. A hypodermic fluid dispensing system according to any preceding claim, wherein the fluid dispenser is a hypodermic needle injector.

23. A hypodermic fluid dispensing system according to any one of claims 1 to 21, wherein the fluid dispenser is a jet injector.

24. A hypodermic fluid dispensing system according to claim 23, wherein the fluid output port is provided in a nozzle which is removable and preferably disposable.

25. A hypodermic fluid dispensing system according to claim 23 or 24, which further includes guard means positioned near the output port for preventing contact between the patient's skin and the output port.

26. A hypodermic fluid dispensing system according to claim 25, wherein the guard means comprises a guard ring.

27. A hypodermic fluid dispensing system according to claim 25 or 26, wherein the outer surface of the guard means has an interrupted pattern to prevent sliding between the dispenser and the patient's skin.

28. A hypodermic fluid dispensing system according to any one of claims 1 to 21 and 23 to 27, wherein the dispenser is a jet injector comprising a collapsible tubular body which forms a chamber and a nozzle provided with a fluid output port capable of communicating with the chamber of the body and which jet injector is either reusable or disposable.

29. A hypodermic fluid dispensing system according to any one of claims 1 to 21 and 23 to 27, wherein the dispenser is a jet injector which comprises a nozzle provided with a fluid output port and a collapsible tubular body which forms a sealed chamber and which is slidably mounted with respect to the nozzle, wherein means are associated with the nozzle for breaching the sealed chamber to allow fluid contained therein to pass out of the output port.

30. A hypodermic fluid dispensing system according to any one of claims 1 to 21 and 23 to 29, wherein the fluid output port of the dispenser is provided in a replaceable nozzle.

31. A hypodermic fluid dispensing system according to any one of claims 1 to 22, wherein the fluid output port is in the form of a hypodermic needle capable of communicating with the chamber of the body, and the dispensing system further comprises means of covering the needle before and after dispersing of the fluid.

32. A hypodermic fluid dispensing system according to claim 31, which further includes means for exposing the needle prior to injection of the fluid.

33. A hypodermic fluid dispensing system according to claim 31 or 32, wherein the means for covering the needle takes the form of a bellows-shaped sheath.

34. A hypodermic fluid dispensing system according to claim 33, wherein the bellows-shaped sheath is capable of containing a liquid which is able to convert to liquid serum lyophilized serum contained in the chamber of the tubular body and wherein the bellows-shaped sheath is capable of communication with the chamber of the tubular body.

35. A hypodermic fluid dispensing system according to any one of claims 31 to 34, which further comprises means for preventing the use of the needle for more than one injection.

36. A hypodermic fluid dispensing system according to claim 35, wherein the prevention means comprises means for crushing the end of the needle to prevent its reuse.

37. A hypodermic fluid dispensing system according to claim 35 or 36, wherein the prevention means comprises a cooperating member provided on the means for applying pressure which is able to cooperate with a corresponding member provided in association with the tubular body so as to prevent withdrawal of the means for applying pressure after use.

38. A hypodermic fluid dispensing system according to claim 35, 36 or 37, wherein the prevention means comprises means for preventing extension of the needle out of the system after use.

**Patentansprüche**

1. Ein hypodermisches Strömungsmittelabgabesystem, das in der Lage ist zur Aufnahme eines Strömungsmittelspenders oder Abgabesystems (Fig. 1; 1A, 5A oder 8A) mit einem zusammenfaltbaren rohrförmigen Körper (100, 122, 500 oder 804), der folgendes bildet: eine Kammer, die in der Lage ist ein hypodermisches Strömungsmittel zu enthalten und einen Strömungsmittelausgangsanschluß (109, 508 oder 807), der in der Lage ist mit der Kammer des Körpers in Verbindung zu stehen, wobei das System folgendes aufweist: Mittel (223, 227 + 113; 510 + 511; oder 703) zum Anlegen von Druck an das eine Ende des Körpers des Spenders, um Strömungsmittel abzugeben, das in der Kammer gehalten ist, und zwar durch den Ausgangsanschluß,
dadurch **gekennzeichnet,**
daß das System weiterhin mit einem Motor (221 oder 1), zum Antreiben der Mittel zum Anlegen von Druck an den Spender vorgesehen ist.

2. Hypodermisches Strömungsmittelabgabesystem nach Anspruch 1, wobei die Kammer anfangs abgedichtet ist, der Ausgangsanschluß von der Kammer isoliert ist und das System weiterhin Mittel aufweist zum selektiven Verbinden des Ausgangsanschlusses mit der Kammer.

3. Hypodermisches Strömungsmittelabgabesystem nach Anspruch 1 oder 2, wobei der Körper ein balgenförmiger zusammenfaltbarer rohrförmiger Körper ist.

4. Hypodermisches Strömungsmittelabgabesystem nach einem oder mehreren der vorhergehenden Ansprüche, wobei die Mittel zum Anlegen von Druck Mittel aufweisen zum wiederholten Speichern von Energie und Mittel aufweisen zum selektiven Freigeben der Energie.

5. Hypodermisches Strömungsmittelabgabesystem nach Anspruch 4, wobei die Mittel zum wiederholten Speichern von Energie eine Feder und Mittel zum Verformen der Feder aufweisen, um Energie in der Feder zu speichern.

6. Hypodermisches Strömungsmittelabgabesystem nach Anspruch 5, wobei die Feder eine Schraubenfeder ist und die Mittel zum Verformen der Feder die Feder zusammendrücken.

7. Hypodermisches Strömungsmittelabgabesystem nach einem der Ansprüche 4, 5 oder 6, wobei das System Mittel aufweist zum Überwachen der gespeicherten Energiemenge und Mittel aufweist zum Beenden eines weiteren Speicherns, wenn die gespeicherte Energie einen vorbestimmten Wert erreicht hat.

8. Hypodermisches Strömungsmittelabgabesystem nach Anspruch 7, das weiterhin manuell einstellbare Mittel zum Erstellen des vorbestimmten Werts aufweisen.

9. Hypodermisches Strömungsmittelabgabesystem nach Anspruch 7 oder 8, wobei die Mittel zum Überwachen der gespeicherten Energie eine Quelle von Bezugsimpulssignalen mit einer ersten Impulsrate aufweisen und eine Quelle von Überwachungsimpulssignalen mit einer Überwachungsimpulsrate, die in direkter Beziehung steht mit der Größe der gespeicherten Energiemenge.

10. Hypodermisches Strömungsmittelabgabesystem nach Anspruch 9, wobei Mittel zum wiederholten Speichern von Energie eine Feder sind und die Mittel zum Überwachen der gespeicherten Energie die Verformung der Feder überwachen und wobei die Impulsrate der Überwachungsimpulssignale Null ist, wenn die Feder in Ruhestellung ist und sie im wesentlichen nicht verformt ist.

11. Hypodermisches Strömungsmittelabgabesystem nach Anspruch 9 oder 10, wobei die Quelle der Überwachungsimpulssignale vorgesehen ist mit einem variablen induktiven Widerstand zur Einstellung der Impulsrate der Überwachungsimpulssignale und wobei der induktive Widerstand als eine Funktion der gespeicherten Energiemenge eingestellt werden kann.

12. Hypodermisches Strömungsmittelabgabesystem nach einem der Ansprühe 8 bis 11, das weiterhin Mittel aufweist zum Anzeigen von Werten, die die Größe der gespeicherten Energie definieren.

13. Hypodermisches Strömungsmittelabgabesystem nach einem oder mehreren der Ansprüche 4 bis 12, das weiterhin folgendes aufweist:
Mittel zum Erzeugen eines Injektionsimpulses jedes Mal, wenn die gespeicherte Energie freigesetzt wurde,
Mittel zum Zählen der Injektionsimpulse, und
Mittel, die auf Ausgangssignale der Zählmittel ansprechen zum Anzeigen, wie oft die Energie freigesetzt wurde.

**14.** Hypodermisches Strömungsmittelabgabesystem nach einem oder mehreren der vorhergehenden Ansprüche, das weiterhin Mittel aufweist zum Überwachen der Funktion des Systems.

**15.** Hypodermisches Strömungsmittelabgabesystem nach Anspruch 14, wobei die Mittel zum Überwachen des Systems folgendes aufweisen:
Zeitsteuermittel,
Mittel zum Definieren und Speichern von Parametern akzeptabler Systemleistungsfähigkeit,
Mittel zum Auswerten der Systemleistungsfähigkeit an Hand der gespeicherten Parameter, und
Mittel zum Anzeigen der Systemfähigkeitsanzeichen.

**16.** Hypodermisches Strömungsmittelabgabesystem nach Anspruch 15, wobei die Mittel zum Überwachen des Systems weiterhin Mittel aufweisen zum Hindern des Systembetriebs, wenn die Systemleistungsfähigkeit außerhalb des annehmbaren Bereichs von gespeicherten Parametern fällt.

**17.** Hypodermisches Strömungsmittelabgabesystem nach Anspruch 15 oder 16, wobei die gespeicherten Parameter einen Bereich von akzeptablen Zeiten für die Fertigstellung einer Injektion aufweisen und die Überwachungsmitel Mittel aufweisen zum Messen der Zeit, die benötigt wird, jede Injektion fertigzustellen und Mittel zum Auswerten der gemessenen Zeit an Hand des Bereichs der akzeptablen Zeiten.

**18.** Hypodermisches Strömungsmittelabgabesystem nach Anspruch 15 oder 16, wobei die gespeicherten Parameter einen Bereich von Werten aufweisen, der eine voraussichtliche Längenänderung des rohrförmigen Körpers während jeder Freigabe von Strömungsmittel definiert und die Überwachungsmittel Mittel aufweisen zum Messen der Längenveränderungen des rohrförmigen Körpers während jeder Freigabe von Strömungsmittel und Mittel zum Auswerten der gemessenen Änderungen an Hand der gespeicherten Parameter.

**19.** Hypodermisches Strömungsmittelabgabesystem nach einem oder mehreren der vorhergehenden Ansprüche, wobei das System aus einem Material gebildet ist, so daß alle Inhalte der Kammer des Körpers sichtbar sind.

**20.** Hypodermisches Strömungsmittelabgabesystem nach einem oder meheren der vorhergehenden Ansprüche, das weiterhin Mittel umfaßt zum Einsetzen des zusammenfaltbare Körpers in das System.

**21.** Hypodermisches Strömungsmittelabgabesystem nach einem oder mehreren der vorhergehenden Ansprüche, das weiterhin elektrische Speichermittel umfaßt zum Aktivieren des Motors.

**22.** Hypodermisches Strömungsmittelabgabesystem nach einem oder mehreren der vorhergehenden Ansprüche, wobei der Strömungsmittelspender ein hypodermischer Nadelinjektor ist.

**23.** Hypodermisches Strömungsmittelabgabesystem nach einem oder mehreren der Ansprüche 1 bis 21, wobei der Strömungsmittelspender ein Düseninjektor ist.

**24.** Hypodermisches Strömungsmittelabgabesystem nach Anspruch 23, wobei der Strömungsmittelausgangsanschluß mit einer Düse versehen ist, die abnehmbar und vorzugsweise wegwerfbar ist.

**25.** Hypodermisches Strömungsmittelabgabesystem nach Anspruch 23 oder 24, das weiterhin Schutzmittel umfaßt, die in der Nähe des Ausgangsanschluß positioniert sind zum Verhindern des Kontakts zwischen der Haut des Patientens und dem Ausgangsanschluß.

**26.** Hypodermisches Strömungsmittelabgabesystem nach Anspruch 25, wobei die Schutzmittel einen Schutzring aufweisen.

**27.** Hypodermisches Strömungsmittelabgabesystem nach Anspruch 25 oder 26, wobei die Außenoberfläche des Schutzmittels ein unterbrochenes Muster aufweist, um das Verrutschen zwischen dem Spender und der Haut des Patienten zu verhindern .

**28.** Hypodermisches Strömungsmittelabgabesystem nach einem oder mehreren der Ansprüche 1 bis 21 und 23 bis 27, wobei der Spender ein Strahl oder Düseninjektor ist, der einen zusammenfaltbaren rohrförmigen Körper aufweist, der eine Kammer bildet und eine Düse vorsieht mit einem Strömungsmittelabgabeanschluß, der in der Lage ist mit der Kammer des Körpers in Verbindung zu stehen und wobei der Düseninjektor entweder wiederverwendbar oder wegwerfbar ist.

29. Hpodermisches Strömungsmittelabgabesystem nach einem oder mehreren der Ansprüche 1 bis 21 und 23 bis 27, wobei der Spender ein Düseninjektor ist, der eine Düse aufweist, die mit einem Strömungsmittelausgangsanschluß vorgesehen ist und einen zusammenleg- oder faltbaren rohrförmigen Körper aufweist, der eine abgedichtete Kammer bildet und der gleitbar bezüglich zur Düse angeordnet ist, wobei Mittel mit der Düse assoziiert sind zum Eindringen in die abgedichtete Kammer, um Strömungsmittel, das darin gehalten wird, zu ermöglichen aus dem Ausgangsanschluß herauszutreten.

30. Hypodermisches Strömungsmittelabgabesystem nach einem oder mehreren der Ansprüche 1 bis 21 und 23 bis 29, wobei der Strömungsmittelausgangsanschluß des Spenders mit einer ersetzbaren Düse vorgesehen ist.

31. Hypodermisches Strömungsmittelabgabesystem nach einem oder mehreren der Ansprüche 1 bis 22, wobei der Strömungsmittelausgangsanschluß in der Form einer hypodermischen Nadel vorgesehen ist, die in der Lage ist mit der Kammer des Körpers in Verbindung zu stehen und wobei das Abgabesystem weiterhin Mittel aufweist zu Abdecken der Nadel vor und nach dem Abgeben des Strömungsmittels.

32. Hypodermisches Strömungsmittelabgabesystem nach Anspruch 31, das weiterhin Mittel umfaßt zum Freilegen der Nadel vor dem Injizieren des Strömungsmittels.

33. Hypodermisches Strömungsmittelabgabesystem nach Anspruch 31 oder 32, wobei die Mittel zum Abdecken der Nadel die Form einer balgenförmigen Schutzhülle annimmt.

34. Hypodermisches Strömungsmittelabgabesystem, nach Anspruch 33, wobei die balgenförmige Schutzhülle in der Lage ist eine Flüssigkeit zu enthalten, die in der Lage ist ein gefriergetrocknetes Serum, das in der Kammer des rohrförmigen Körpers enthalten ist, in flüssiges Serum umzuwandeln, und wobei die balgenförmige Schutzhülle in der Lage ist, mit der Kammer des rohrförmigen Körpers in Verbindung zu stehen.

35. Hypodermisches Strömungsmittelabgabesystem nach einem der Ansprüche 31 bis 34, das weiterhin Mittel aufweist zum Verhindern der Verwendung der Nadel für mehr als eine Injektion.

36. Hypodermisches Strömungsmittelabgabesystem nach Anspruch 35, wobei die Verhinderungsmittel Mitel aufweisen zum Zerbechen des Endes der Nadel, um ihre Wiederverwendung zu verhindern.

37. Hypodermisches Strömungsmittelabgabesystem nach Anspruch 35 oder 36, wobei die Verhinderungsmittel ein Zusammenarbeitsglied aufweisen, das an den Mitteln zum Anlegen von Druck vorgesehen ist und das in der Lage ist mit einem entsprechenden Glied zusammenzuarbeiten, das zusammen mit dem rohrförmigen Körper vorgesehen ist, um das Herausziehen der Mittel zum Anlegen von Druck nach der Verwendung zu verhindern.

38. Hypodermisches Strömungsmittelabgabesystem nach Anspruch 35, 36 oder 37, wobei die Verhinderungsmittel Mittel aufweisen zum Verhindern des sich aus dem System Heraauserstrecken der Nadel nach der Verwendung.

## Revendications

1. Un système de distribution de fluide hypodermique, recevant un distributeur de fluide (figures 1, 1A, 5A ou 8A) ayant un corps tubulaire rétractable (100, 122, 500 ou 804) qui forme une chambre capable de contenir un fluide hypodermique, et un orifice de sortie de fluide (109, 508 ou 507) qui peut communiquer avec la chambre du corps, ce système comportant :

   des moyens (223, 227 + 113; 510 + 511; ou 703) destinés à appliquer une pression à une extrémité du corps du distributeur, de façon à évacuer à travers l'orifice de sortie le fluide qui est contenu dans la chambre,

   caractérisé en ce que le système comporte également un moteur (221 ou 1), prévu pour entraîner les moyens destinés à appliquer une pression au distributeur.

**2.** Un système de distribution de fluide hypodermique selon la revendication 1, dans lequel la chambre est initialement fermée hermétiquement, l'orifice de sortie est isolé de la chambre, et le système comprend en outre des moyens destinés à mettre sélectivement l'orifice de sortie en communication avec la chambre.

**3.** Un système de distribution de fluide hypodermique selon la revendication 1 ou 2, dans lequel le corps est un corps tubulaire rétractable en forme de soufflet.

**4.** Un système de distribution de fluide hypodermique selon l'une quelconque des revendications précédentes, dans lequel les moyens destinés à appliquer une pression comprennent des moyens qui sont conçus pour emmagasiner de l'énergie de façon répétée, et des moyens qui sont conçus pour libérer sélectivement cette énergie.

**5.** Un système de distribution de fluide hypodermique selon la revendication 4, dans lequel les moyens destinés à emmagasiner de l'énergie de façon répétée comprennent un ressort,des moyens étant prévus pour déformer le ressort de façon à emmagasiner de l'énergie dans celui-ci.

**6.** Un système de distribution de fluide hypodermique selon la revendication 5, dans lequel le ressort est un ressort hélicoïdal, les moyens de déformation du ressort comprimant ce ressort.

**7.** Un système de distribution de fluide hypodermique selon l'une quelconque des revendications 4, 5 ou 6, dans lequel le système comprend des moyens destinés à contrôler la quantité d'énergie qui est emmagasinée, et des moyens destinés à arrêter le stockage d'énergie lorsque cette dernière atteint une valeur prédéterminée.

**8.** Un système de distribution de fluide hypodermique selon la revendication 7, comprenant en outre des moyens réglables manuellement pour fixer la valeur prédéterminée.

**9.** Un système de distribution de fluide hypodermique selon la revendication 7 ou 8, dans lequel les moyens destinés à contrôler l'énergie emmagasinée comprennent une source de signaux d'impulsions de référence ayant une première cadence d'impulsions et une source de signaux de contrôle ayant une cadence d'impulsions de contrôle qui est directement liée à la valeur de la quantité d'énergie emmagasinée.

**10.** Un système de distribution de fluide hypodermique selon la revendication 9, dans lequel les moyens destinés à emmagasiner de l'énergie de façon répétée consistent en un ressort, et les moyens destinés à contrôler l'énergie emmagasinée contrôlent la déformation du ressort, la cadence d'impulsions des signaux de contrôle étant égale à zéro lorsque le ressort est au repos et n'est pratiquement pas déformé.

**11.** Un système de distribution de fluide hypodermique selon la revendication 9 ou 10, dans lequel la source d'impulsions de contrôle est munie d'une inductance variable pour fixer la cadence d'impulsions des signaux de contrôle, et l'inductance peut être réglée en fonction de la quantité d'énergie emmagasinée.

**12.** Un système de distribution de fluide hypodermique selon l'une quelconque des revendications 8 à 11, comprenant en outre des moyens qui sont destinés à visualiser des valeurs qui définissent la quantité d'énergie emmagasinée.

**13.** Un système de distribution de fluide hypodermique selon l'une quelconque des revendications 4 à 12, comprenant en outre des moyens qui sont destinés à produire une impulsion d'injection chaque fois que l'énergie emmagasinée a été libérée, des moyens destinés à compter l'impulsion d'injection, et des moyens qui réagissent aux signaux de sortie des moyens de comptage en visualisant le nombre de fois que l'énergie a été libérée.

**14.** Un système de distribution de fluide hypodermique selon l'une quelconque des revendications précédentes, comprenant en outre des moyens destinés à contrôler les performances du système.

**15.** Un système de distribution de fluide hypodermique selon la revendication 14, dans lequel les moyens destinés à contrôler le système comprennent des moyens de mesure du temps, des moyens destinés à définir et à enregistrer des paramètres de performances acceptables du système, des moyens destinés à évaluer les performances du système sur la base des paramètres enregistrés, et des moyens destinés à visualiser des indications de performances du système.

**16.** Un système de distribution de fluide hypodermique selon la revendication 15, dans lequel les moyens destinés à contrôler le système comprennent en outre des moyens destinés à empêcher le fonctionnement du système lorsque les performances de celui-ci tombent à l'extérieur d'une plage acceptable des paramètres enregistrés.

**17.** Un système de distribution de fluide hypodermique selon la revendication 15 ou 16, dans lequel les paramètres enregistrés comprennent une plage de temps acceptables pour l'accomplissement d'une injection, les moyens de contrôle comprenant des moyens pour mesurer le temps nécessaire pour effectuer chaque injection et des moyens pour évaluer le temps mesuré, sur la base de la plage de temps acceptable.

**18.** Un système de distribution de fluide hypodermique selon la revendication 15 ou 16, dans lequel les paramètres enregistrés comprennent une plage de valeurs qui définissent un changement prévu de longueur du corps tubulaire pendant chaque expulsion de fluide, les moyens de contrôle comprenant des moyens pour mesurer les changements de longueur du corps tubulaire pendant chaque expulsion de fluide et des moyens pour évaluer les changements mesurés, sur la base des paramètres enregistrés.

**19.** Un système de distribution de fluide hypodermique selon l'une quelconque des revendications précédentes, dans lequel le système est formé avec un matériau tel que le contenu de la chambre du corps soit visible.

**20.** Un système de distribution de fluide hypodermique selon l'une quelconque des revendications précédentes, comprenant en outre des moyens prévus pour introduire le corps rétractable dans le système.

**21.** Un système de distribution de fluide hypodermique selon l'une quelconque des revendications précédentes, comprenant en outre des moyens de stockage d'électricité pour actionner le moteur.

**22.** Un système de distribution de fluide hypodermique selon l'une quelconque des revendications précédentes, dans lequel le distributeur de fluide est un injecteur à aiguille hypodermique.

**23.** Un système de distribution de fluide hypodermique selon l'une quelconque des revendications 1 à 21, dans lequel le distributeur de fluide est un injecteur à jet.

**24.** Un système de distribution de fluide hypodermique selon la revendication 23, dans lequel l'orifice de sortie de fluide est formé dans un embout amovible et de préférence jetable.

**25.** Un système de distribution de fluide hypodermique selon la revendication 23 ou 24, comprenant en outre des moyens de garde placés près de l'orifice de sortie pour empêcher le contact entre la peau du patient et l'orifice de sortie.

**26.** Un système de distribution de fluide hypodermique selon la revendication 25, dans lequel les moyens de garde consistent en un anneau.

**27.** Un système de distribution de fluide hypodermique selon la revendication 25 ou 26, dans lequel la surface extérieure des moyens de garde présente une configuration interrompue, pour empêcher un glissement entre le distributeur et la peau du patient.

**28.** Un système de distribution de fluide hypodermique selon l'une quelconque des revendications 1 à 21 et 23 à 27, dans lequel le distributeur est un injecteur à jet, comprenant un corps tubulaire rétractable qui forme une chambre et un embout muni d'un orifice de sortie de fluide capable de communiquer avec la chambre du corps, l'injecteur à jet étant réutilisable ou jetable.

**29.** Un système de distribution de fluide hypodermique selon l'une quelconque des revendications 1 à 21 et 23 à 27, dans lequel le distributeur est un injecteur à jet qui comprend une buse munie d'un orifice de sortie de fluide et un corps tubulaire rétractable qui forme une chambre fermée hermétiquement et qui est monté de façon coulissante par rapport à l'embout, dans lequel des moyens sont associés à l'embout pour former une ouverture dans la chambre fermée hermétiquement pour permettre au fluide contenu dans cette dernière de sortir par l'orifice de sortie.

**30.** Un système de distribution de fluide hypodermique selon l'une quelconque des revendications 1 à 21 et 23 à 29, dans lequel l'orifice de sortie de fluide du distributeur est placé dans un embout remplaçable.

31. Un système de distribution de fluide hypodermique selon l'une quelconque des revendications 1 à 22, dans lequel l'orifice de sortie de fluide se présente sous la forme d'une aiguille hypodermique capable de communiquer avec la chambre du corps, le système de distribution comprenant en outre des moyens destinés à recouvrir l'aiguille avant et après la distribution du fluide.

32. Un système de distribution de fluide hypodermique selon la revendication 31, comprenant en outre des moyens destinés à mettre l'aiguille à nu avant l'injection du fluide.

33. Un système de distribution de fluide hypodermique selon la revendication 31 ou 32, dans lequel les moyens destinés à recouvrir l'aiguille consistent en une gaine en forme de soufflet.

34. Un système de distribution de fluide hypodermique selon la revendication 33, dans lequel la gaine en forme de soufflet peut contenir un liquide qui est capable de convertir en sérum liquide du sérum lyophilisé contenu dans la chambre du corps tubulaire, et dans lequel la gaine en forme de soufflet peut communiquer avec la chambre du corps tubulaire.

35. Un système de distribution de fluide hypodermique selon l'une quelconque des revendications 31 à 34, comprenant en outre des moyens destinés à empêcher l'utilisation d'une aiguille pour plus d'une injection.

36. Un système de distribution de fluide hypodermique selon la revendication 35, dans lequel les moyens destinés à empêcher la réutilisation d'une aiguille comprennent des moyens destinés à écraser l'extrémité de l'aiguille pour empêcher cette réutilisation.

37. Un système de distribution de fluide hypodermique selon la revendication 35 ou 36, dans lequel les moyens destinés à empêcher la réutilisation de l'aiguille comprennent un élément associé, formé sur les moyens destinés à appliquer une pression, capable de coopérer avec un élément correspondant formé en association avec le corps tubulaire, de façon à empêcher l'extraction des moyens d'application de pression, après utilisation.

38. Un système de distribution de fluide hypodermique selon la revendication 35, 36 ou 37, dans lequel les moyens destinés à empêcher la réutilisation de l'aiguille comprennent des moyens qui sont destinés à empêcher l'extension de l'aiguille hors du système après utilisation.

Fig. 1

Fig. 2A

Fig. 2B

Fig. 1A

Fig. 2AA

Fig. 2BB

Fig. 3

Fig. 4

426

436

IC SET

427 IC SET 1
428 IC SET 2
IC SET 3
IC SET 4
430

429

400          402
$F_b$
406
404
408

410          412
$F_{r2}$
414
416          418

420
C    $Q_7$  $F_{d2}$
D  SR  $\overline{Q}_7$

422
IN    $D_1$
R     $D_N$
$\overline{Q}_2$

PULSE DECODER, COUNTER AND DISPLAY
424              425
BT

MULTIPLE INNOCULATION CARRIAGE ADVANCE MECH.

1                                                    5              6
ENERGY SOURCE  →  ENERGY STORAGE  →  PRESSURE RESTRAINT  →  BELLOWS SERUM CHAMBER  →  FLOW ORIFICE

2                    3
4
TRIGGER RELEASE

7
PRESSURE SENSOR

8
VOLUME SENSOR

9
VELOCITY SENSOR

13
SYSTEM POWER FUNCTIONS
P1    P2

10
IC SET

11
PROCESSOR & DECISION LOGIC

CUT-OFF CONTROL

12
MONITOR DISPLAY AND/OR WARNING

Fig. 6

Fig. 5A

Fig. 5B

Fig. 5C

Fig. 5D

Fig. 5E

# Fig. 7

Fig. 8A

Fig. 8B